# EUROPÄISCHE PATENTSCHRIFT

(11) **EP 0 462 357 B1**
(45) Veröffentlichungstag und Bekanntmachung des Hinweises auf die Patenterteilung: **04.05.1994**
(21) Anmeldenummer: 91101825.7
(22) Anmeldetag: 09.02.1991
(51) Int. Cl.: A61F 2/36

(54) **Oberschenkelteil einer Hüftgelenkendoprothese**
Femur part of a HP joint endoprothesis
Partie fémorale d'une endoprothèse pour l'articulation de la hanche

(30) Priorität: 20.06.1990 DE 9006893 U
(43) Veröffentlichungstag der Anmeldung: 27.12.1991
(73) Patentinhaber: Howmedica GmbH, D-24232 Schönkirchen (DE)
(72) Erfinder: Prof. Dr. Täger, Karl Heinrich, W-2316 Gauting (DE); Harder, Hans E., W-2302 Probsteierhagen (DE)
(74) Vertreter: Patentanwälte Hauck, Graalfs, Wehnert, Döring, Siemons

(56) Entgegenhaltungen:
- EP-A- 0 243 585
- EP-A- 0 289 922
- DE-A- 2 851 598
- DE-U- 9 006 893
- TECHNISCHE MITTEILUNGEN KRUPP Nr. 1, Juni 1989, Seiten 37-42, Essen, DE; G. BENSMANN et al.: "On the problem of cementless fixation of endoprostheses"
- idem

## Beschreibung

Die Neuerung bezieht sich auf einen Oberschenkelteil einer Hüftgelenkendoprothese nach dem Oberbegriff des Anspruchs 1, wie er aus dem DE-U-87 12 607 bekannt ist.

Aus diesem DE-U-87 12 607 ist ein Oberschenkelteil bekanntgeworden, bei dem der Schaft als Hohlkörper ausgebildet ist und mehrere Öffnungen aufweist, die den Schafthohlraum mit den Seiten des Schafts verbinden. Die Öffnungen sind vorzugsweise an der frontalen und dorsalen Seite geformt und können verschiedene Konfigurationen haben. Die Ausbildung des Schaftes als Hohlkörper hat den Vorteil, daß er mit körpereigener autologer oder heterologer Spongiosa gefüllt werden kann, wodurch das Einheilen der Prothese im Femur durch eine echte knöcherne Verbindung erfolgt, die eine sichere Verankerung ermöglicht, die auch über einen längeren Zeitraum ungefährdet ist. Bei derartigen Prothesen wird das spongiose Material über die seitlichen Öffnungen eingefüllt, was sich für den Chirurgen etwas schwierig gestaltet. Insbesondere ist es schwierig, eine gleichmäßig verteilte und verdichtete Masse im Inneren des Schaftes einzubringen.

Der Neuerung liegt die Aufgabe zugrunde, das Einbringen der Spongios in die Prothese zu vereinfachen und zu verbessern.

Diese Aufgabe wird durch die Merkmale des Anspruchs 1 gelöst.

Bei der neuerungsgemäßen Prothese ist der kragenlose Endabschnitt des Schaftes am Übergang zum Hals massiv ausgeführt. Lateral vom Hals ist eine Bohrung im massiven Abschnitt geformt, deren Achse annähernd mit der Achse des Schafthohlraums zusammenfällt. Über die Bohrung kann Spongiosa eingefüllt werden, und zwar auch nach dem Einsetzen des Schaftes im Femur. Die Lage bzw. Anordnung der Bohrung ermöglicht, die eingefüllte Spongiosa mit Hilfe eines geeigneten Werkzeugs in ausreichendem Maße zusammenzupressen. Ein gewisses Komprimieren des eingefüllten Materials ist erwünscht, um vor dem Einsetzen des Schaftes ein Herausfallen des Materials zu vermeiden und anschließend für optimale Wachstumsbedingungen zu sorgen.

Wie erwähnt, kann die Spongiosa mit Hilfe eines Werkzeugs von oben komprimiert werden. Alternativ sieht eine Ausgestaltung der Neuerung vor, daß die Bohrung ein Innengewinde aufweist zur Aufnahme einer Schraube. Die Schraube dient als Stopfen, der beim Eindrehen in die Bohrung das darunter befindliche Material zusammendrückt.

Bei einer anderen Ausgestaltung der Neuerung weist die Bohrung am oberen Ende einen Abschnitt größeren Durchmessers auf zur Aufnahme eines Schraubenkopfes. Der Schraubenkopf kann mit dem in der Bohrung gebildeten Absatz zusammenwirken und dadurch gleichzeitig als Schlagfläche dienen, um den Schaft während der Operation in den Femur einzutreiben. Die Bildung einer separaten Schlagfläche hat den Vorteil, daß das Material des Schaftes beim Eintreiben mit dem Schlagwerkzeug nicht deformiert oder in anderer Weise beschädigt wird, was unter Umständen ungünstige Folgen für die Lebensdauer hat.

Nach einer weiteren Ausgestaltung der Neuerung weist der Schraubenkopf einen zylindrischen Bund und oberhalb des Bundes Werkzeugangriffsflächen auf, vorzugsweise einen Sechskant, wobei der Bund passend von dem erweiterten Bohrungsabschnitt aufgenommen ist und die Angriffsflächen etwas kleiner in ihren Außenabmessungen sind als der Durchmesser des Bundes. Insbesondere wenn die Werkzeugangriffsflächen etwas aus der Bohrung herausstehen, kann die Schraube beim implantierten Schaft herausgeschraubt werden, um einen Zugang zum Inneren des Schafthohlraums zu erhalten. Außerdem kann dann in die Bohrung ein Ausziehelement eingeschraubt werden, um den Schaft zum Zwecke einer Reoperation wieder aus dem Femur zu entfernen.

Beim neuerungsgemäßen Oberschenkelteil können die länglichen Öffnungen eine ovale Form haben. Sämtliche Öffnungen weisen eine Fase am Rand auf dergestalt, daß sich die Öffnung zum Schafthohlraum verjüngt. Bei einer Ausgestaltung der Neuerung sind die Durchmesser aller Kreisöffnungen bzw. die Länge aller ovalen Öffnungen gleich, wobei die Breite der ovalen Öffnungen dem Durchmesser der Kreisöffnungen entsprechen kann. Der konische Eintrittsbereich hat den Vorteil, daß die Spongiosa leichter eingefüllt werden kann. Er bildet eine Art Trichter. Die Fase setzt darüber hinaus die Spannungsspitzen herab, die bei Belastung auftreten könnten.

Im proximalen Bereich sind zwei Öffnungsreihen vorgesehen, von denen jeweils eine eine Kreisöffnung und eine ovale Öffnung aufweist, wobei medial die ovale Öffnung und lateral die kreisrunde Öffnung oben liegt. Auf diese Weise wird eine günstige Verteilung der beiden unterschiedlichen Öffnungstypen erhalten mit günstigen Einwachsmöglichkeiten von Knochensubstanz und bei ausreichender Stabilität des Schaftes auch im proximalen Bereich.

Ein Ausführungsbeispiel der Neuerung wird nachfolgend anhand von Zeichnungen näher erläutert.
- Fig. 1: zeigt eine Seitenansicht einer Endoprothese nach der Neuerung.
- Fig. 2: zeigt eine weitere Seitenansicht der Prothese nach Fig. 1 in Richtung Pfeil 2.
- Fig. 3: zeigt einzelne Schnitte durch die Prothese nach Fig.1.
- Fig. 4: zeigt eine ähnliche Seitenansicht wie Fig. 1,jedoch teilweise im Schnitt.
- Fig. 5: zeigt vergrößert einen Schraubstopfen für die Prothese nach Fig. 1 bzw. 4.
- Fig. 6: zeigt eine Endansicht des Schraubstopfens nach Fig. 5 in Richtung Pfeil 6.
- Fig. 7: zeigt einen Schnitt durch einen Teil einer Endoprothese nach der Neuerung.

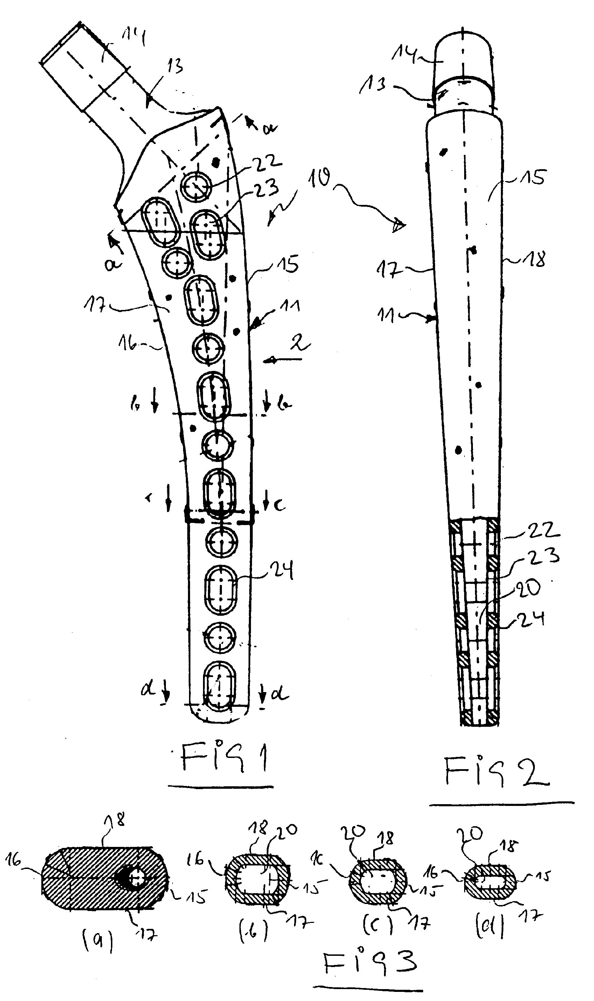

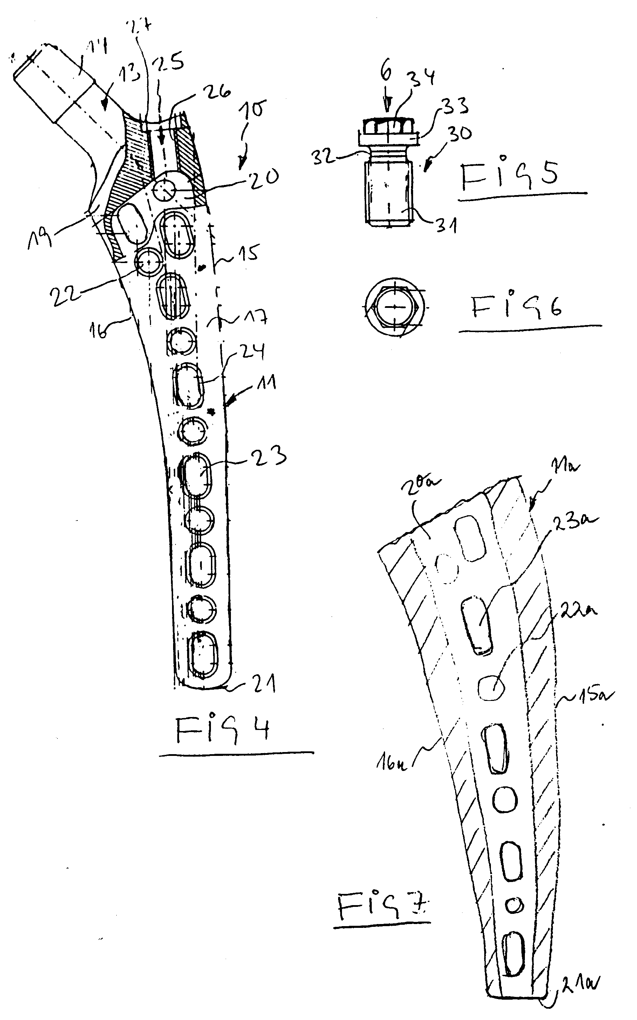

In Fig. 1 und 2 ist ein Oberschenkelteil 10 dargestellt. Er besteht aus einem relativ schlanken hohlen Schaft 11, der am proximalen Ende ohne Kragen in einen sich konisch verjüngenden Hals 13 übergeht, der am Ende einen Konus 14 aufweist zur Aufnahme einer nicht gezeigten Gelenkkugel, die einen entsprechenden passenden Innenkonus aufweist. Der Winkel zwischen Schaft- und Halsachse beträgt 135°.

Der Schaft 11 weist eine laterale Seite 15 und eine mediale Seite 16 auf. Die sichtbare frontale Seite ist mit 17 bezeichnet, während die dorsale mit 18 bezeichnet ist (siehe Fig. 2 bzw. 3). Aus Fig. 3 ist zu entnehmen, daß frontale Seite 17 und dorsale Seite 18 annähernd parallel verlaufen und daß die mediale Seite 16 und die laterale Seite 15 kreisbogenförmige Kontur im Querschnitt aufweisen.

Der Schaft 11 weist einen Hohlraum 20 auf, der sich kanalförmig durch die Länge des gesamten Schaftes erstreckt. Der Hohlraum 20 ist zum abgerundeten distalen Ende 21 geöffnet. Nach proximal erstreckt er sich bis zu einem massiven oberen Endabschnitt 19 des Schaftes 11, wobei sich die Decke des Hohlraums 20 annähernd im rechten Winkel zur Achse des Halses 13 erstreckt. Die Dicke des Abschnitts 19 hängt von der gewünschten Festigkeit ab, mit der der Hals 13 gegenüber dem Schaft 11 abgestützt werden soll.

Die frontale Seite 17 und die dorsale Seite 18 des Schaftes 11 sind mit Kreisöffnungen 22 und ovalen Öffnungen 23 versehen. Eine erste Reihe von Öffnungen 22, 23, die sich abwechseln, erstreckt sich entlang der Mittellinie des Schaftes 11 bzw. parallel dazu (proximal). Eine zweite Reihe aus einer Kreisöffnung 22 und einer ovalen Öffnung 23 erstreckt sich annähernd parallel zur ersten Reihe, wobei in der ersten Reihe proximal zuoberst eine ovale Öffnung und darunter eine Kreisöffnung vorgesehen sind, während die Reihenfolge in der parallelen Reihe umgekehrt ist. Wie ferner zu erkennen, liegt die zweite Reihe annähernd auf einer Linie mit den darunter befindlichen Öffnungen 22, 23, wobei diese Linie den lateralen Bereich der Schulter zwischen Schaft 11 und Hals 13 mit dem distalen Ende 21 annähernd mittig verbindet. Diese Linie fällt andererseits annähernd mit der Achse des Femurs zusammen.

Der Durchmesser der Kreisöffnungen 22 ist für alle gleich. Die Breite der ovalen Öffnungen 23 entspricht dem Durchmesser der Kreisöffnungen 22. Die Länge der ovalen Öffnungen entspricht dem doppelten Durchmesser der Kreisöffnungen. Die Längsachse der ovalförmigen Öffnungen 23 im mittleren und distalen Bereich fällt mit der Längsachse des Schaftes 12 zusammen. Die Ränder der Öffnungen 22, 23 sind mit einer Fase 24 versehen, wodurch sich ein trichterartiger Eingangsbereich zum Hohlraum 20 ergibt. Die Form und Anordnung der Öffnungen 22, 23 ist derart, daß ein optimales Einwachsen des Knochens in den Schaft erreicht wird bei ausreichender, gleichmäßiger Stabilität des Schaftes. Die Fasen 24 ermöglichen ein leichteres seitliches Einfüllen von Spongiosa und setzen Spannungsspitzen herab. Die Wanddicke des Schaftes im Bereich des Hohlraums 20 ist annähernd konstant, so daß sich der Hohlraum 20 nach distal kontinuierlich konisch verjüngt.

Auf der lateralen Seite des Halses 13 ist im massiven Abschnitt 19 eine Bohrung 25 geformt, deren Achse annähernd mit der Längsachse des Schaftes 11 bzw. des Hohlraums 20 zusammenfällt. Die Bohrung 25 weist einen Gewindeabschnitt 26 auf, der sich bis zum Hohlraum 20 erstreckt. Am oberen Ende geht der Bohrungsabschnitt 26 in einen im Durchmesser erweiterten Abschnitt 27 über. Die Bohrung 25 dient zur Aufnahme eines Schraubstopfens 30, der in den Figuren 5 und 6 näher dargestellt ist. Der Schraubstopfen 30 weist einen Gewindeschaft 31 auf, der am oberen Ende in einen im Durchmesser kleineren Halsabschnitt 32 übergeht, an den sich seinerseits ein kreisförmiger zylindrischer Bund 33 anschließt. Oberhalb des Bunds 33 befindet sich ein Sechskantkopf 34, dessen Maximaldurchmesser kleiner ist als der Durchmesser des Bunds 33. Der Schraubstopfen 30 wird in die Gewindebohrung 25 eingeschraubt, nachdem der Hohlraum 20 mit Spongiosa aufgefüllt worden ist. Das Einfüllen findet über die Öffnungen 22 bzw. 23 statt sowie auch über die Gewindebohrung 25. Es sei noch erwähnt, daß die Öffnungen 22, 23 an den einander gegenüberliegenden Seiten zueinander ausgerichtet sind. Mit Hilfe des Schraubstopfens 30 wird das in der Bohrung 25 und darunter befindliche Spongiosamaterial komprimiert. Zu diesem Zweck steht der Stopfen 30 etwas über das untere Ende des Bohrungsabschnitts 26 über, wenn der Bund 33 passend vom erweiterten Bohrungsabschnitt 27 aufgenommen ist. Der Sechskantkopf 34 dient zum Einschrauben des Stopfens 30. Er steht etwas über die Oberseite des proximalen Endes des Schaftes 11 über, so daß sowohl beim Einschrauben als auch beim Ausschrauben des Stopfens 30 dieser von einem Schraubenschlüssel erfaßt werden kann. Der Kopf 34 dient darüber hinaus als Schlagfläche beim Eintreiben der Prothese 10 in einen Femur. Soll der Femur zum Beispiel zwecks Reoperation entfernt werden, wird der Stopfen 30 herausgeschraubt und statt dessen ein anderes Element eingeschraubt, das einen Auszugabschnitt aufweist, beispielsweise eine Öse oder dergleichen, um die Prothese 10 herausschlagen zu können.

Der gezeigte Prothesenteil ist als Gußteil geformt, zum Beispiel aus Titan oder aus einer Chrom-Kobalt-Legierung (Vitallium).

Bei der Ausführungsform nach Fig. 7 ist zu erkennen, daß der teilweise dargestellte Schaft 11a in der Lateral-Medial-Ebene eine von oben nach unten abnehmende Wanddicke hat, also z.B. oben eine Dicke von 4 mm und unten von 3 mm. Die Wanddicke in der Anterior-Posterior-Ebene ist dagegen über die Länge des Schaftes 11a konstant.

## Patentansprüche

1. Oberschenkelteil (10) einer Hüftgelenkendoprothese mit einem als Hohlkörper ausgebildeten Schaft (11), der mehrere Öffnungen (22,23) an der frontalen (17) und dorsalen (18) Seite aufweist, die mit dem Schafthohlraum (20) verbunden sind, wobei kreisrunde Öffnungen (22) mit länglichen (23) abwechseln, und einem Hals (13), an dem entweder einteilig ein kugelförmiger Kopf oder ein Aufnahmeabschnitt (14) für einen kugelförmigen Kopf angeformt ist, dadurch gekennzeichnet, daß der kragenlose Endabschnitt (19) des Schaftes (11) am Übergang zum Hals (13) massiv ausgeführt ist und lateral vom Hals eine bis in den Schafthohlraum reichende Bohrung (25) im massiven Endabschnitt (19) geformt ist, deren Achse annähernd mit der Achse des Schafthohlraums (20) zusammenfällt.

2. Oberschenkelteil nach Anspruch 1, dadurch gekennzeichnet, daß die Bohrung (25) ein Innengewinde (26) aufweist zur Aufnahme eines Schraubstopfens (30).

3. Oberschenkelteil nach Anspruch 2,dadurch gekennzeichnet, daß die Bohrung (25) am äußeren Ende einen Bohrungsabschnitt (27) größeren Durchmessers aufweist zur Aufnahme eines Schraubenkopfes (33, 34).

4. Oberschenkelteil nach Anspruch 3,dadurch gekennzeichnet, daß der Schraubenkopf einen zylindrischen Bund (33) und oberhalb des Bundes (33) Werkzeugangriffsflächen (34), vorzugsweise einen Sechskant, aufweist, wobei der Bund (33) passend von dem erweiterten Bohrungsabschnitt (27) aufgenommen ist und die Außenabmessungen der Angriffsflächen (34) kleiner sind als der Durchmesser des Bundes (33).

5. Oberschenkelteil nach Anspruch 4,dadurch gekennzeichnet, daß der Kopf des Schraubstopfens (30) etwas aus der Bohrung (25) heraussteht.

6. Oberschenkelteil nach einem der Ansprüche 3 bis 5, dadurch gekennzeichnet, daß der Schaft (31) des Schraubstopfens (30) etwas in den Schafthohlraum (20) hineinsteht, wenn der Kopf sich im Bohrungsabschnitt mit dem größeren Durchmesser befindet.

7. Oberschenkelteil nach einem der Ansprüche 1 bis 6, dadurch gekennzeichnet, daß die länglichen Öffnungen (23) eine ovale Form haben und alle Öffnungen (22, 23) am Rand eine Fase (24) aufweisen dergestalt, daß sie einen sich konisch verjüngenden Eintrittsbereich bilden.

8. Oberschenkelteil nach Anspruch 7,dadurch gekennzeichnet, daß der Durchmesser aller Kreisöffnungen (22, 23) bzw. die Länge aller ovalen Öffnungen (23) gleich groß ist.

9. Oberschenkelteil nach Anspruch 7 oder 8, dadurch gekennzeichnet, daß im proximalen Bereich zwei Öffnungsreihen vorgesehen sind, die jeweils eine kreisrunde und eine ovale Öffnung aufweisen, wobei medial die obere Öffnung oval und lateral die obere kreisrund ist.

10. Oberschenkelteil nach Anspruch 1, dadurch gekennzeichnet, daß die Wanddicke des Schaftes (11a) in der Lateral-Medial-Ebene von proximal nach distal allmählich abnimmt, während die Wanddicke in der Anterior-Posterior-Ebene über die Länge des Schaftes (11a) annähernd konstant ist.

## Claims

1. A femur part (10) of a hip joint endoprosthesis, comprising a stem (11) formed as a hollow body and having a plurality of openings (22, 23) on its front (17) and dorsal (18) side, said openings being connected to the hollow space (20) of said stem and with circular (22) and elongated openings (23) being alternatingly arranged, and a neck (13) to which either a spherical head or a receiving portion (14) for a spherical head is formed as one piece, characterized in that the collarless end portion (19) of said stem (11), where said stem (11) transforms to said neck (13), is solid, and a bore (25), extending to the hollow space of said stem, is formed in said solid end portion (19) lateral of said neck, the axis of said bore being approximately aligned with the axis of the hollow space (20) of said stem.

2. The femur part of claim 1, wherein said bore (25) includes an inner thread (26) for the accommodation of a threaded plug (30).

3. The femur part of claim 2, wherein said bore (25) has a bore portion (27) of a larger diameter at the extreme end thereof for the receipt of a screw head (33, 34).

4. The femur part of claim 3, wherein said screw head has a cylindrical flange (33) and tool-engaging surfaces (34) above said flange (33), preferably a hexagon, said flange (33) being matingly accommodated by the enlarged bore portion (27) and the outer dimensions of said tool-engaging surfaces (34) being smaller than the diameter of said flange (33).

5. The femur part of claim 4, wherein said head of said threaded plug (30) slightly extends beyond said bore (25).

6. The femur part of any of the claims 3 to 5, wherein said shank (31) of said threaded plug (30) slightly extends into said hollow space (20) of said stem when said screw head is within said bore portion of a larger diameter.

7. The femur part of any of the claims 1 to 6, wherein said elongated openings (23) are oval and the edges of all of said openings (22, 23) have a chamfer (24) to form a conically constricting entrance portion.

8. The femur part of claim 7, wherein the diameter of all of said openings (22, 23) or the length of all of said oval openings (23) is equal.

9. The femur part of claim 7 or 8, wherein two rows of said openings are provided in the proximal area, each including one circular and one oval opening with the upper opening of the medial row being oval while the upper opening of the lateral row being circular.

10. The femur part of claim 1, wherein in the lateral- medial-plane the wall thickness of said stem (11a) gradually reduces downwardly while in the anterior-posterior-plane, the wall thickness is approximately constant throughout the length of said stem (11a).

## Revendications

1. Partie fémorale (10) d'une prothèse interne d'articulation de la hanche, comportant un fût (11) façonné en forme de corps creux, qui présente plusieurs orifices (22, 23) sur la face frontale (17) et la face dorsale (18), lesquels sont en communication avec la cavité intérieure du fût (20), selon une alternance d'orifices ronds (22) et d'orifices oblongs (23); et un col (13) auquel s'adapte suivant les cas une tête sphérique ou une pièce (14) destinée à accueillir une tête sphérique, caractérisé en ce que la partie terminale (19) dépourvue de collerette, de ce fût (11) est massive dans la section où elle se raccorde au col (13), et en ce que, latéralement par rapport au col, il est pratiqué, dans la partie terminale massive (19), un forage (25) qui atteint la cavité intérieure du fût, et dont l'axe coïncide approximativement avec l'axe de la cavité intérieure (20) du fût.

2. Partie fémorale selon la revendication 1, caractérisée en ce que le forage (25) présente un filetage intérieur (26) destiné à accueillir un bouchon fileté (30).

3. Partie fémorale selon la revendication 2, caractérisée en ce que le forage (25) présente à son extrémité externe une section (27) de diamètre supérieur, destinée à accueillir une tête de vis (33, 34).

4. Partie fémorale selon la revendication 3, caractérisée en ce que la tête de vie présente une collerette cylindrique (33), et au-dessus de la collerette (33) une section à pans, de préférence hexagonale (34), de prise pour un outil, ce qui permet d'insérer la collerette (33), qui s'ajuste à la section élargie du forage (27), et que les dimensions extérieures de la section hexagonale (34) sont inférieures au diamètre de la collerette (33).

5. Partie fémorale selon la revendication 4, caractérisée par le fait que la tête du bouchon fileté (30) s'avance un peu hors du forage (25).

6. Partie fémorale selon l'une des revendications 3 à 5, caractérisée en ce que le corps (31) du bouchon fileté (30) pénètre légèrement dans la cavité intérieure du fût (20), quand la tête se trouve dans la partie du forage dont le diamètre est supérieur.

7. Partie fémorale selon l'une des revendications 1à 6, caractérisée en ce que les orifices oblongs (23) ont une forme ovale et que tous les orifices (22, 23) présentent un bord biseauté (24), de telle sorte qu'ils constituent des zones d'entrées se rétrécissant de manière conique.

8. Partie fémorale selon la revendication 7,caractérisée en ce que les diamètres de tous les orifices (22, 23), de même que les grands axes de tous les orifices ovales (23) ont des dimensions analogues.

9. Partie fémorale selon la revendication 7 ou la revendication 8, caractérisée par le fait que, dans la zone proximale, il est prévu deux rangées d'orifices qui présentent respectivement une orifice rond et un orifice ovale, disposés de telle sorte que dans la région moyenne, l'orifice supérieur est ovale, et dans la région latérale l'orifice supérieur est rond.

10. Partie fémorale selon la revendication 1, caractérisé par le fait que l'épaisseur de la paroi du fût (11a) diminue progressivement dans le plan frontal médian, de l'extrémité proximale à l'extrémité distale, alors que l'épaisseur de la paroi est à peu près constante dans le plan antéro-postérieur sur toute la longueur du fût (11a).
